# EUROPEAN PATENT APPLICATION

(11) **EP 4 821 438 A1**
(43) Date of publication of application: **14.10.2026**
(21) Application number: 25169513.6
(22) Date of filing: 09.04.2025
(51) Int. Cl.: A61B 5/367

(54) **DECREMENT EVOKED POTENTIAL MAPPING**

(71) Applicant: St. Jude Medical, Cardiology Division, Inc., St. Paul, MN 55117 (US); University Hospitals Coventry and Warwickshire NHS Trust, Coventry CV2 2DX (GB)
(72) Inventor: DHANJAL, Tarv, Coventry, CV2 2DX (GB); WINTER, James, Shrewsbury, SY2 5UD (GB)
(74) Representative: Mathys & Squire

(57) **Abstract**

A computer-implemented method of decrement evoked potential mapping is disclosed herein. The method comprises obtaining a plurality of EGM signals, wherein each EGM signal comprises S1 and S2 stimuli; obtaining an indication of the location of each obtained EGM signal in the heart; determining S1 and S2 EGM durations from each obtained EGM signal; determining the degree of decrement based on the determined S1 and S2 EGM durations; and mapping the degree of decrement to a 3D mapped geometry of the heart.

## Description

### Field of the invention

The present disclosure relates to a computer-implemented method and system for automated decrement evoked potential mapping.

### Background

Ventricular tachycardia (VT) ablation approaches based on high-density mapping, which enable the rapid acquisition of thousands of mapping points in order to delineate slow conduction zones, have been widely adopted.

Substrate ablation is often based on late potentials (LPs) and local abnormal ventricular activities (LAVAs). However, the conducting channels critical to VT induction/maintenance need not be active during sinus rhythm mapping. Decrement-evoked potential (DeEP) mapping is a method to unmask these critical channels and to identify those with the potential for unidirectional block to initiate tachycardia.

The concept of DeEP mapping was born from the understanding that the initiation of a re-entrant VT circuit is dependent upon delayed or decremental conduction through a critical zone of tissue. If this slow conduction occurs adjacent to a site of unidirectional block, then a re-entrant circuit may develop.

DeEP mapping identifies the components of diseased myocardium that are more likely to be critical to initiating or maintaining the VT circuit. This is achieved by delivering an extra stimulus and comparing the local activation delay during steady-state conditions (S1) and after the extra stimulus (S2). A decrement of more than about 10 ms in the local electrogram (EGM) component has been proven to be highly specific for the detection of the critical sites of the VT circuit and thus provides a mechanistic target for substrate-based ablation (Bhaskaran et al., Decrement Evoked Potential Mapping to Guide Ventricular Tachycardia Ablation; Elucidating the Functional Substrate, Arrhythmia & Electrophysiology Review 2020;9(4):211-8).

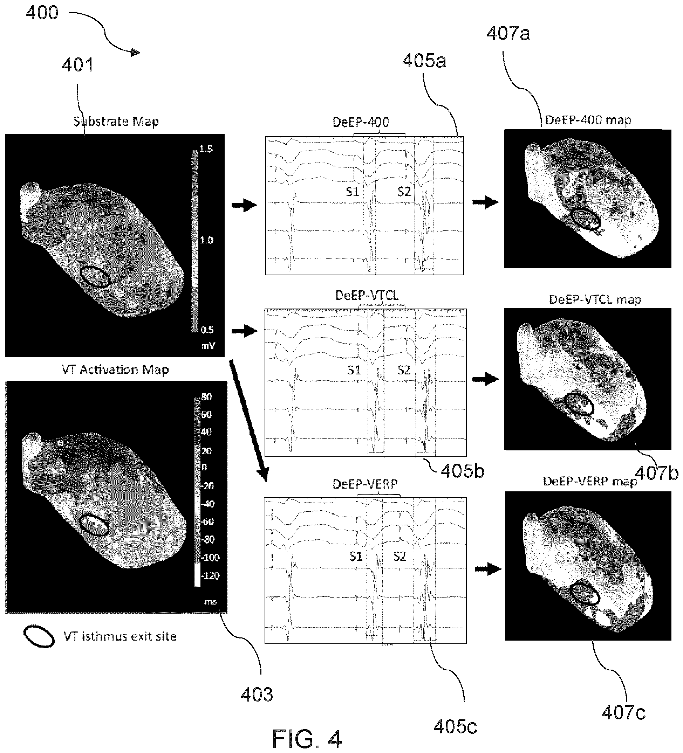

Al-Sheikhli et al, "Optimization of decrementing evoked potential mapping for functional substrate identification in ischaemic ventricular tachycardia ablation", Europace (2023) 00, 1-3, describes a study where omnipolar EGMs were exported for bespoke offline analysis to create DeEP maps (shown in FIG. 4) based on differences in S1 and S2 EGM duration. The DeEP maps were constructed with decrement thresholds ranging from 10 to 50 ms in 10 ms increments for three S2 coupling intervals. Percentage DeEP map areas were calculated as a proportion of the total mapped area. The VT exit site was used for comparison with the DeEP maps. The definition of DeEP was altered from the standard >10 ms to multiple thresholds of >10 to >50 ms in 10 ms increments. For all three DeEP maps, sensitivity decreased, while specificity increased, for larger thresholds. The optimal threshold where the sensitivity and specificity curves crossed was >20 ms. The study found that DeEP signals are best defined by decrement >20 ms; and (ii) DeEP areas are co-localized more accurately to the VT exit site with a 400 ms S2.

During mapping, intracardiac electrograms (EGM) provide specific information that the cardiac electrophysiologist is required to rapidly interpret during the course of a procedure in order to perform an effective ablation.

While manual mapping may be possible for a single stimulus, manual construction of DeEP maps using multi-electrode catheters (such as the Advisor^{™} HD Grid Mapping Catheter, Sensor Enabled^{™} (SE) (Abbott Laboratories; Abbott Park, Illinois)) would be extremely time consuming (Bhaskaran et al, Decrement Evoked Potential Mapping to Guide Ventricular Tachycardia Ablation; Elucidating the Functional Substrate, Arrhythmia & Electrophysiology Review 2020;9(4):211-8) and practically extremely challenging for the clinician in real time for signals with temporal and spatial cohesion.

### Summary of the invention

Aspects of the invention are as set out in the independent claims and optional features are set out in the dependent claims. Aspects of the invention may be provided in conjunction with each other and features of one aspect may be applied to other aspects.

In a first aspect there is provided a computer-implemented method of decrement evoked potential mapping. The method comprises obtaining a plurality of EGM signals, wherein each EGM signal comprises S1 and S2 stimuli; obtaining an indication of the location of each obtained EGM signal in the heart; determining S1 and S2 EGM duration from each obtained EGM signal; determining the degree of decrement based on the determined S1 and S2 EGM duration; and mapping the degree of decrement to a 3D mapped geometry of the heart.

Determining the degree of decrement based on the determined S1 and S2 EGM duration may comprise performing an arithmetic function of the S1 and S2 EGM duration, such as an arithmetic subtraction of the S2 duration from the S1 duration.

The method may further comprise mapping the geometry of the heart. Mapping the geometry of the heart may comprise forming a geometric model of the cardiac surface, and/or an electrophysiology, EP, map. Forming an EP map may comprise receiving a plurality of electrophysiology, EP, data points at an electroanatomical mapping system, wherein each EP data point of the plurality of EP data points includes electrophysiological signals; computing, with the electroanatomical mapping system, a metric for each EP data point using the electrophysiological signals for the respective EP data point; and outputting, with the electroanatomical mapping system, a graphical representation that facilitates visualization of the heart based on the electrophysiological signals for the respective EP data point.

The electrophysiological signals may be measured by a plurality of electrode pairs.

Obtaining a plurality of EGM signals may comprise obtaining EGM signals using a grid-patterned electrode. The EGM signals may be bipolar or omnipolar.

The method may further comprise obtaining a template beat that contains both the S1 and S2 and corresponding local EGM signals, for example one cycle of ventricular tachycardia, VT. Obtaining a plurality of EGM signals may comprise recognizing, by pattern matching, when the stimuli have been delivered.

The current EGM signal may match the template beat based on a user defined matching score/threshold. If the correct coupling intervals are used, it is possible to fit both stimuli within S1S2 two beat waveform, pattern matching can be performed on that. In other words, if the correct coupling interval is selected, both stimuli can be delivered within a single window of interest, such that morphological matching can be performed on an electrogram that includes both stimuli, instead of having to do two matches on the S1 and S2 stimuli, respectively.

Obtaining an indication of the location of each obtained EGM signal in the heart may comprise obtaining an indication of the location of each obtained EGM signal in the heart at the time the S1 and S2 stimuli have been delivered. Obtaining a location may comprise determining the location of an electrode (such as a pair of electrodes) at the time the S1 and S2 stimuli have been delivered.

Determining S1 and S2 EGM duration from each obtained EGM signal may comprise at least one of:
1. determining the time from the start of the S1 signal to the end of the S2 beat (the *end of the S2 beat may be the last deflection*);
2. determining when the EGM signals cross a selected voltage threshold;
3. analysing the frequency of the EGM signal to determine the S1 and S2 duration; and
4. analysing the energy distribution of the EGM signal.

Mapping the degree of decrement to a 3D mapped geometry of the heart may comprise mapping the degree of decrement to a 3D mapped geometry of the heart only in the event that the determined degree of decrement is above a selected threshold level degree of decrement.

The method may further comprise generating a colour map by interpolating the values of the obtained degree of decrement to the vertices of the 3D mapped geometry of the heart.

The degree of decrement may be calculated based on an arithmetic function of the S2 EGM and S1 EGM durations, for example by the arithmetic subtraction of the S2 EGM and S1 EGM duration.

The method may further comprise applying the S1 and S2 extra-stimulus stimuli, wherein the S2 extra-stimulus is at a user-selected coupling interval. The S1 and S2 extra-stimulus stimuli may be delivered by any electrode, but typically they may be delivered from a catheter at the right ventricle (RV) apex, or from a fixed device in the RV or left ventricle (LV).

In another aspect there is provided a system for decrement evoked potential, DeEP, mapping. The system comprises a mapping and visualization module configured to: receive a plurality of EGM signals, wherein each EGM signal of the plurality of EGM signals comprises at least S1 and S2 stimuli; compute a metric for the degree of decrement at each electrode pair as a function of electrode location; and output a graphical representation that facilitates visualization of the degree of decrement as a function of location.

The system may comprise a high-density grid catheter, and the electrophysiological signals may be measured by the high-density grid catheter.

The system or mapping and visualization module may be configured to obtain a template beat that contains both the S1 and S2 and corresponding local EGM signals.

Computing a metric for the degree of decrement may comprise recognizing, by pattern matching, when the stimuli have been delivered. Computing a metric for the degree of decrement further may comprise determining S1 and S2 EGM duration from each obtained EGM signal, and determining the degree of decrement based on an arithmetic function of the S1 and S2 duration.

Determining the S1 and S2 duration may comprise at least one of:
determining the time from the start of the S1 signal to the end of the S2 beat (for example, the end of the S2 beat may be the last deflection);
determining when the EGM signals cross a selected voltage threshold;
analysing the frequency of the EGM signal to determine the S1 and S2 duration.; and
analysing the energy distribution of the EGM signal.

The mapping and visualization module may be configured to generate a colour map by interpolating the values of the obtained degree of decrement to the vertices of the 3D mapped geometry of the heart. The mapping and visualization module may be configured to scale the map according to the user's requirements, for example so that areas of decrement (e.g., >10ms) are displayed as a specific colour on the 3D map. In some examples, the DeEP signals could be represented by a glyph that highlights signals above a defined threshold. These glyphs can be displayed on top of any map (e.g. voltage map).

The system may further be configured to applying the S1 and S2 extra-stimulus stimuli, wherein the S2 extra-stimulus is at a user-selected coupling interval.

In another aspect there is provided a computer readable non-transitory storage medium comprising a program for a computer configured to cause a processor to perform the method of the aspect described above.

### Drawings

Embodiments of the disclosure will now be described, by way of example only, with reference to the accompanying drawings, in which:
FIG. 1 is a schematic diagram of an exemplary electroanatomical mapping system that can be used in connection with aspects of the present disclosure;
FIG. 2 depicts an exemplary catheter that can be used in connection with aspects of the present disclosure;
FIG. 3 shows an example electrogram (EGM) illustration;
FIG. 4 shows example substrate and VT activation maps, EGM signals and DeEP maps;
FIG. 5 shows a flowchart of a representative method that may be performed by an electroanatomical mapping system for automated decrement evoked potential mapping;
FIG. 6 shows a flowchart of another representative method that may be performed by an electroanatomical mapping system for automated decrement evoked potential mapping; and
FIG. 7 shows a flowchart of another representative method that may be performed by an electroanatomical mapping system for automated decrement evoked potential mapping.

### Specific description

Intracardiac electrophysiology devices typically include a catheter having a proximal end and a distal end that includes a plurality of electrodes. Signals sensed by the plurality of electrodes are utilized by electrophysiology systems to perform a number of functions, including determining the location of the electrodes within the patient's body and sensing electrograms associated with adjacent cardiac tissue. This information can be utilized to understand electrical activity of the heart and identify the origins and pathways of abnormal heart rhythms such as arrythmias.

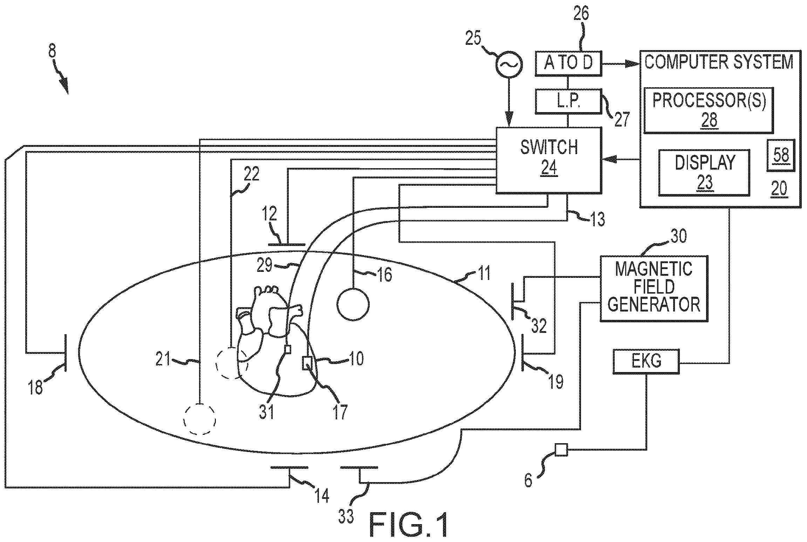

**FIG. 1** shows a schematic diagram of an exemplary electroanatomical mapping system 8 for conducting cardiac electrophysiology studies by navigating a cardiac catheter and measuring electrical activity occurring in a heart 10 of a patient 11 and three-dimensionally mapping the electrical activity and/or information related to or representative of the electrical activity so measured. System 8 can be used, for example, to create an anatomical model of the patient's heart 10 using one or more electrodes. System 8 can also be used to measure electrophysiology data at a plurality of points along a cardiac surface and store the measured data in association with location information for each measurement point at which the electrophysiology data was measured, for example to create a diagnostic data map of the patient's heart 10.

As one of ordinary skill in the art will recognize, system 8 determines the location, and in some aspects the orientation, of objects, typically within a three-dimensional space, and expresses those locations as position information determined relative to at least one reference. This is referred to herein as "localization". For simplicity of illustration, the patient 11 is depicted schematically as an oval. In the embodiment shown in **FIG. 1****,** three sets of surface electrodes (e.g., patch electrodes) are shown applied to a surface of the patient 11, defining three generally orthogonal axes, referred to herein as an x-axis, a y-axis, and a z-axis. In other embodiments the electrodes could be positioned in other arrangements, for example multiple electrodes on a particular body surface. As a further alternative, the electrodes do not need to be on the body surface, but could be positioned internally to the body.

In **FIG. 1****,** the x-axis surface electrodes 12, 14 are applied to the patient along a first axis, such as on the lateral sides of the thorax region of the patient (e.g., applied to the patient's skin underneath each arm) and may be referred to as the Left and Right electrodes. The y-axis electrodes 18, 19 are applied to the patient along a second axis generally orthogonal to the x-axis, such as along the inner thigh and neck regions of the patient, and may be referred to as the Left Leg and Neck electrodes. The z-axis electrodes 16, 22 are applied along a third axis generally orthogonal to both the x-axis and the y-axis, such as along the sternum and spine of the patient in the thorax region, and may be referred to as the Chest and Back electrodes. The heart 10 lies between these pairs of surface electrodes 12/14, 18/19, and 16/22.

An additional surface reference electrode (e.g., a "belly patch") 21 provides a reference and/or ground electrode for the system 8. The belly patch electrode 21 may be an alternative to a fixed intra-cardiac electrode 31, described in further detail below. It should also be appreciated that, in addition, the patient 11 may have most or all of the conventional electrocardiogram ("ECG" or "EKG") system leads in place. In certain embodiments, for example, a standard set of 12 ECG leads may be utilized for sensing electrocardiograms on the patient's heart 10. This ECG information is available to the system 8 (e.g., it can be provided as input to computer system 20). Insofar as ECG leads are well understood, and for the sake of clarity in the figures, only a single lead 6 and its connection to computer 20 is illustrated in FIG. 1.

A representative catheter 13 having at least one electrode 17 is also shown. This representative catheter electrode 17 is referred to as the "roving electrode", "moving electrode", or "measurement electrode". Typically, multiple electrodes 17 on catheter 13, or on multiple such catheters, will be used. In one embodiment, for example, the system 8 may comprise sixty-four electrodes on twelve catheters disposed within the heart and/or vasculature of the patient. In other embodiments, system 8 may utilize a single catheter that includes multiple (e.g., eight) splines, each of which in turn includes multiple (e.g., eight) electrodes.

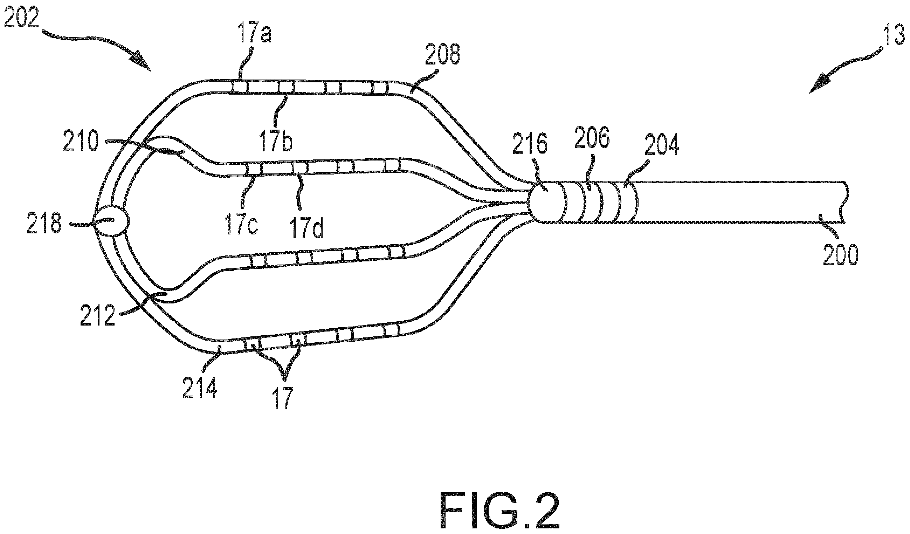

The foregoing embodiments are merely exemplary, however, and any number of electrodes and/or catheters may be used. For example, for purposes of this disclosure, a segment of an exemplary multi-electrode catheter, and in particular an HD grid catheter, is shown in **FIG.** 2. HD grid catheter 13 includes a catheter body 200 coupled to a paddle 202. Catheter body 200 can further include first and second body electrodes 204, 206, respectively. Paddle 202 can include a first spline 208, a second spline 210, a third spline 212, and a fourth spline 214, which are coupled to catheter body 200 by a proximal coupler 216 and to each other by a distal coupler 218. In one embodiment, first spline 208 and fourth spline 214 can be one continuous segment and second spline 210 and third spline 212 can be another continuous segment. In other embodiments, the various splines 208, 210, 212, 214 can be separate segments coupled to each other (e.g., by proximal and distal couplers 216, 218, respectively). It should be understood that HD catheter 13 can include any number of splines; the four-spline arrangement shown in **FIG. 2** is merely exemplary.

As described above, splines 208, 210, 212, 214 can include any number of electrodes 17; in **FIG. 2****,** sixteen electrodes 17 are shown arranged in a four-by-four array. It should also be understood that electrodes 17 can be evenly and/or unevenly spaced, as measured both along and between splines 208, 210, 212, 214.

As those of ordinary skill in the art will appreciate, electrodes 17 carried in the distal portion of catheter 13 can also be used to measure unipolar electrograms (e.g., between any electrode 17 and a patch electrode 12, 14, 16, 18, 19, 21, or 22). Unipolar electrograms have the advantage of being orientation-independent (that is, a given electrode 17 will measure substantially the same unipolar electrogram regardless of the orientation of catheter 13 relative to the cardiac surface). On the other hand, unipolar electrograms often include not only the component of interest (e.g., a near-field cardiac activation component), but also various far-field noise components. These far-field noise components include, but are not limited to, far-field cardiac activation components, powerline noise components, patient respiration components, patient motion components, and cardiac motion components. Unipolar electrograms also ignore local directional information in the form of the ion currents that produce electrograms.

As those of ordinary skill in the art will recognize, any two neighbouring electrodes 17 define a bipole. Thus, the 16 electrodes 17 on catheter 13 define a total of 42 bipoles 12 along splines (e.g., between electrodes 17a and 17b, or between electrodes 17c and 17d), 12 across splines (e.g., between electrodes 17a and 17c, or between electrodes 17b and 17d), and 18 diagonally between splines (e.g., between electrodes 17a and 17d, or between electrodes 176 and 17c).

Any bipole can, in turn, be used to generate a bipolar electrogram according to techniques that will be familiar to those of ordinary skill in the art. Bipolar electrograms are less susceptible to far-field noise than unipolar electrograms and incorporate directional information along their axes. Those of ordinary skill in the art will recognize, however, that bipolar electrograms are orientation-dependent (that is, they will change as the orientation of catheter 13 relative to the cardiac surface changes).

Bipolar electrograms can be combined (e.g., linearly combined) to generate electrograms, again including activation timing information, in any direction of the plane of catheter 13 to create "omnipolar electrograms" or "virtual bipolar electrograms". In other words, bipolar electrograms can be combined to generate electrograms for any orientation of catheter 13 relative to the cardiac surface without physically changing the orientation of catheter 13. These "omnipolar electrograms" or "virtual bipolar electrograms" can be generated by computing an E-field loop for a clique of electrodes. The term "clique" refers to a group or cluster of neighboring or closely-spaced electrodes 17 on catheter 13. Each individual electrode 17 within a clique can be used to measure a unipolar electrogram; those of ordinary skill in the art will be familiar with various techniques suitable for defining a representative unipolar electrogram for the clique as a whole (*e.g*., an average of three individual unipolar electrograms measured by the three electrodes of the clique). Likewise, each pair of electrodes 17 within a clique can be used to measure a bipolar electrogram. E-field loops can be computed from these various unipolar and bipolar electrograms, and for omnipolar electrograms generated therefrom. United States patent nos. 10,758,137 and 10,194,994; international patent application publication no. WO 2015/130824; and Deno et al., Orientation-Independent Catheter-Based Characterization of Myocardial Activation, IEEE Transactions on Biomedical Engineering, Vol. 64, No. 5, 1067-1077 (May 2017) ("Deno"), which are hereby incorporated by reference as though fully set forth herein, disclose details of computing an E-field loop for a clique of electrodes on a HD grid catheter. These are referred to herein as "omnipoles" or "virtual bipoles". Omnipolar technology (OT) can generate electroanatomic voltage maps with orientation-independent electrograms. These omnipolar electrograms can be thought of as the bipolar electrogram that would be seen by an "omnipole" or "virtual bipole" having its "omnipole orientation" or "virtual bipole orientation" at a particular angle relative to the cardiac anatomy (e.g., the cardiac surface).

In any event, catheter 13 can be used to simultaneously collect a plurality of electrophysiology data points for the various unipoles, bipoles and omnipoles defined by electrodes 17 thereon, with each such electrophysiology data point including both localization information (e.g., position and orientation of a unipole; position and orientation of a selected bipole or electrode clique) and a corresponding electrogram signal (*e.g*., unipolar, bipolar, and/or omnipolar electrograms). For purposes of illustration, methods according to the instant disclosure will be described with reference to individual electrophysiology data points collected by catheter 13. It should be understood, however, that the teachings herein can be applied, in serial and/or in parallel, to multiple electrophysiology data points collected by catheter 13.

Catheter 13 (or multiple such catheters) are typically introduced into the heart and/or vasculature of the patient via one or more introducers and using familiar procedures. Indeed, various approaches to introduce catheter 13 into a patient's heart, such as transseptal approaches, will be familiar to those of ordinary skill in the art, and therefore need not be further described herein.

Since each electrode 17 lies within the patient, location data may be collected simultaneously for each electrode 17 by system 8. Similarly, each electrode 17 can be used to gather electrophysiological data from the cardiac surface (e.g., surface electrograms). The person skilled in the art will be familiar with various modalities for the acquisition and processing of electrophysiology data points (including, for example, both contact and non-contact electrophysiological mapping), such that further discussion thereof is not necessary to the understanding of the techniques disclosed herein. Likewise, various techniques familiar in the art can be used to generate a graphical representation of a cardiac geometry and/or of cardiac electrical activity from the plurality of electrophysiology data points. Moreover, insofar as the person skilled in the art will appreciate how to create electrophysiology maps from electrophysiology data points, the aspects thereof will only be described herein to the extent necessary to understand the present disclosure.

Returning now to **FIG. 1****,** in some embodiments, optional fixed reference electrode 31 (e.g., attached to a wall of the heart 10) is shown on a second catheter 29. For calibration purposes, this electrode 31 may be stationary (e.g., attached to or near the wall of the heart) or disposed in a fixed spatial relationship with the roving electrodes (e.g., electrodes 17), and thus may be referred to as a "navigational reference" or "local reference". The fixed reference electrode 31 may be used in addition or alternatively to the surface reference electrode 21 described above. In many instances, a coronary sinus electrode or other fixed electrode in the heart 10 can be used as a reference for measuring voltages and displacements; that is, as described below, fixed reference electrode 31 may define the origin of a coordinate system.

Each surface electrode is coupled to a multiplex switch 24, and the pairs of surface electrodes are selected by software running on a computer 20, which couples the surface electrodes to a signal generator 25. Alternately, switch 24 may be eliminated and multiple (e.g., three) instances of signal generator 25 may be provided, one for each measurement axis (that is, each surface electrode pairing). As the catheter 13 and electrodes 17 navigate within the body, the electrodes 17 sense voltages. The voltages sensed by the electrodes 17 are communicated to the computer system 20 via switch 24, low-pass filter 27, and analog-to-digital converter 26, which processes the sensed signal.

The computer 20 may comprise, for example, a conventional general-purpose computer, a special-purpose computer, a distributed computer, or any other type of computer. The computer 20 may comprise one or more processors 28, such as a single central processing unit ("CPU"), or a plurality of processing units, commonly referred to as a parallel processing environment, which may execute instructions to practice the various aspects described herein.

Generally, three nominally orthogonal electric fields are generated by a series of driven and sensed electric dipoles (e.g., surface electrode pairs 12/14, 18/19, and 16/22) in order to realize catheter navigation in a biological conductor. Alternatively, these orthogonal fields can be decomposed and any pairs of surface electrodes can be driven as dipoles to provide effective electrode triangulation. Likewise, the electrodes 12, 14, 18, 19, 16, and 22 (or any number of electrodes) could be positioned in any other effective arrangement for driving a current to or sensing a current from an electrode in the heart. For example, multiple electrodes could be placed on the back, sides, and/or belly of patient 11. Additionally, such non-orthogonal methodologies add to the flexibility of the system. For any desired axis, the potentials measured across the roving electrodes resulting from a predetermined set of drive (source-sink) configurations may be combined algebraically to yield the same effective potential as would be obtained by simply driving a uniform current along the orthogonal axes.

Thus, any two of the surface electrodes 12, 14, 16, 18, 19, 22 may be selected as a dipole source and drain with respect to a ground reference, such as belly patch 21, while the unexcited electrodes measure voltage with respect to the ground reference. The roving electrodes 17 placed in the heart 10 are exposed to the field from a current pulse and are measured with respect to ground, such as belly patch 21. In practice the catheters within the heart 10 may contain more or fewer electrodes than the sixteen shown, and each electrode potential may be measured. As previously noted, at least one electrode may be fixed to the interior surface of the heart to form a fixed reference electrode 31, which is also measured with respect to ground, such as belly patch 21, and which may be defined as the origin of the coordinate system relative to which system 8 measures positions. Data sets from each of the surface electrodes, the internal electrodes, and the virtual electrodes may all be used to determine the location of the roving electrodes 17 within heart 10.

The measured voltages may be used by system 8 to determine the location in three-dimensional space of the electrodes inside the heart, such as roving electrodes 17 relative to a reference location, such as reference electrode 31. That is, the voltages measured at reference electrode 31 may be used to define the origin of a coordinate system, while the voltages measured at roving electrodes 17 may be used to express the location of roving electrodes 17 relative to the origin. In some embodiments, the coordinate system is a three-dimensional (x, y, z) Cartesian coordinate system, although other coordinate systems, such as polar, spherical, and cylindrical coordinate systems, are contemplated.

As should be clear from the foregoing discussion, the data used to determine the location of the electrode(s) within the heart is measured while the surface electrode pairs impress an electric field on the heart. The electrode data may also be used to create a respiration compensation value used to improve the raw location data for the electrode locations as described, for example, in U.S. Pat. No. 7,263,397, which is hereby incorporated herein by reference in its entirety. The electrode data may also be used to compensate for changes in the impedance of the body of the patient as described, for example, in U.S. Pat. No. 7,885,707, which is also incorporated herein by reference in its entirety.

Therefore, in one representative embodiment, system 8 first selects a set of surface electrodes and then drives them with current pulses. While the current pulses are being delivered, electrical activity, such as the voltages measured with at least one of the remaining surface electrodes and in vivo electrodes, is measured and stored. Compensation for artifacts, such as respiration and/or impedance shifting, may be performed as indicated above.

In aspects of the disclosure, system 8 can be a hybrid system that incorporates both impedance-based (e.g., as described above) and magnetic-based localization capabilities. Thus, for example, system 8 can also include a magnetic source 30, which is coupled to one or more magnetic field generators. In the interest of clarity, only two magnetic field generators 32 and 33 are depicted in FIG. 1, but it should be understood that additional magnetic field generators (e.g., a total of six magnetic field generators, defining three generally orthogonal axes analogous to those defined by patch electrodes 12, 14, 16, 18, 19, and 22) can be used without departing from the scope of the present teachings . Likewise, those of ordinary skill in the art will appreciate that, for purposes of localizing catheter 13 within the magnetic fields so generated, can include one or more magnetic localization sensors (e.g., coils).

In some embodiments, system 8 is the EnSite^{™} X, EnSite^{™} Velocity^{™} or EnSite Precision^{™} cardiac mapping and visualization system of Abbott Laboratories. Other localization systems, however, may be used in connection with the present teachings, including for example the RHYTHMIA HDX^{™} mapping system of Boston Scientific Corporation (Marlborough, Mass.), the CARTO^{®} navigation and location system of Biosense Webster, Inc. (Irvine, Calif.), the AURORA^{®} system of Northern Digital Inc. (Waterloo, Ontario), Sterotaxis, Inc.'s NIOBE^{®} Magnetic Navigation System (St. Louis, Mo.), as well as MediGuide^{™} Technology from Abbott Laboratories .

The localization and mapping systems described in the following patents (all of which are hereby incorporated by reference in their entireties) can also be used with the present invention: U.S. Pat. Nos. 12,167,917; 6,990,370; 6,978,168; 6,947,785; 6,939,309; 6,728,562; 6,640,119; 5,983,126; and 5,697,377.

Aspects of the disclosure generally relate to generating electrophysiology maps, and in particular to DeEP maps using multi-electrode catheters. Such maps may be useful to identify the components of diseased myocardium that are more likely to be critical to initiating or maintaining the VT circuit. Graphical representations of such DeEP maps can also be output, for example on display 23.

System 8 can therefore include a mapping and visualization module 58. Advantageously, visualization of such DeEP maps can aid a practitioner in identifying the components of diseased myocardium that are more likely to be critical to initiating or maintaining the VT circuit.

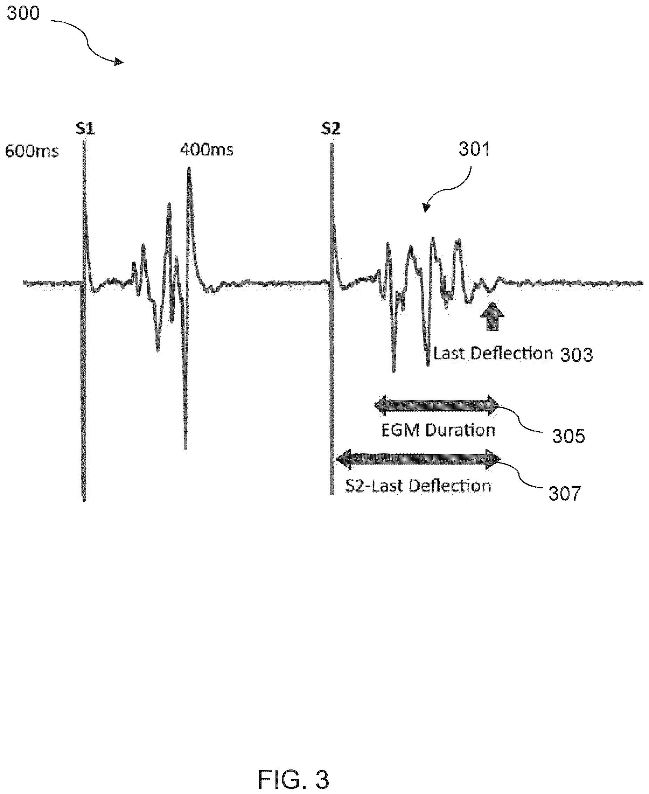

DeEP mapping requires the analysis of signals generated from two pacing stimuli, the second at a shorter coupling interval than the first. These stimuli are denoted S1 and S2. The general principle is to measure the length/duration of the electrogram resulting from the S2 stimulus and compare this arithmetically with the duration of the electrogram resulting from the S1. An example of an electrogram comprising the S1 and S2 stimuli is shown in **FIG. 3****.**

**FIG. 3** shows an example electrogram (EGM) illustration 300 reproduced from Heart Rhythm, Vol 18, No 5, May 2021, of a pacing drive train (S1) at 600 ms and closely coupled extrastimulus (S2) (ventricular effective refractory period [VERP] + 30 ms) to reveal fractionation 301 before a last deflection 303, indicative of functional conduction delay. EGM duration 305 and time from S2-last deflection 307 are increased.

The duration of the signal, measured in milliseconds, may be measured between two features of the signal within a defined window of interest. An example is that of the first and last time a signal within the window crosses a defined voltage threshold, but other metrics such as energy-based or frequency-based timing annotations could be used. Signals that decrement, and are therefore of interest clinically, will exhibit a larger S2-S1 interval.

During a mapping procedure, the physician will move a mapping catheter (e.g., Advisor^{™} HD Grid) into different locations and deliver a pacing train; typically consisting of several S1 stimuli at a fixed pacing interval and a single S2 stimulus. Conventionally, it is necessary for the physician to review each signal on the lab's EP system in turn and to assess signals that have decremented. The physician then instructs the mapper to place lesions on the relevant poles as a marker of regions of interest. By moving the mapping catheter around the chamber, it is possible to build up a 3D record of regions of tissue that exhibit decremental properties, information which can be used by the physician, alongside other inputs, to define an ablation strategy. An example of such a 3D record is shown in **FIG. 4** which shows the DeEP maps 400 of *Al-Sheikhli et al.* described above, constructed with decrement thresholds ranging from 10 to 50 ms in 10 ms increments for three S2 coupling intervals (400 ms (DeEP-400), clinical VT cycle length (DeEO-VTCL), and VERP + 20 ms (DeEP-VERP)).

**FIG. 4** shows a substrate map 401 and a VT activation map 403 produced using the mapping process described above. FIG. 4 shows three electrogram traces 405a-c each containing multiple electrogram signals constructed with decrement thresholds ranging from 10 to 50 ms in 10 ms increments for three S2 coupling intervals (400 ms (DeEP-400), clinical VT cycle length (DeEO-VTCL), and VERP + 20 ms (DeEP-VERP)). FIG. 4 also shows three corresponding DeEP maps 407a-c for the three different S2 coupling intervals. Within the three electrogram traces 405a-c it is also possible to see lines indicating the beginning and ends of the S1 and S2 EGM signals indicating the respective S1 and S2 durations.

There as some notable disadvantages with this approach because:
1. With high-density mapping, the physician must manually review several thousand points which is laborious and prone to error; and
2. Often by the time the signals have been reviewed, the catheter may have moved and so the labelled 3D tags may not be accurately located.

Embodiments of the disclosure involve optimization of the DeEP mapping protocol by automating the above processes. Embodiments of the disclosure involve the system 8 and mapping and visualization module 58 being set to automatically collect data that matches several user-defined criteria, process the signals within the window of interest, and generate several colour maps that represent features of the analyzed signals (e.g., peak-to-peak voltage, timing relative to a fixed timing reference, etc.).

However, conventional systems do not currently allow the user to make measurements of signal length/duration, nor to arithmetically compare measurements made from two different beats/stimuli. As noted above, measuring the length/duration of the electrogram resulting from the S2 stimulus and comparing this arithmetically with the duration of the electrogram resulting from the S1 as a function of location provides the DeEP map (or "subtraction map"). This cannot be provided using conventional systems. Furthermore, embodiments of the disclosure facilitate multiple EGM signals being analyzed in parallel, something again not possible with conventional systems.

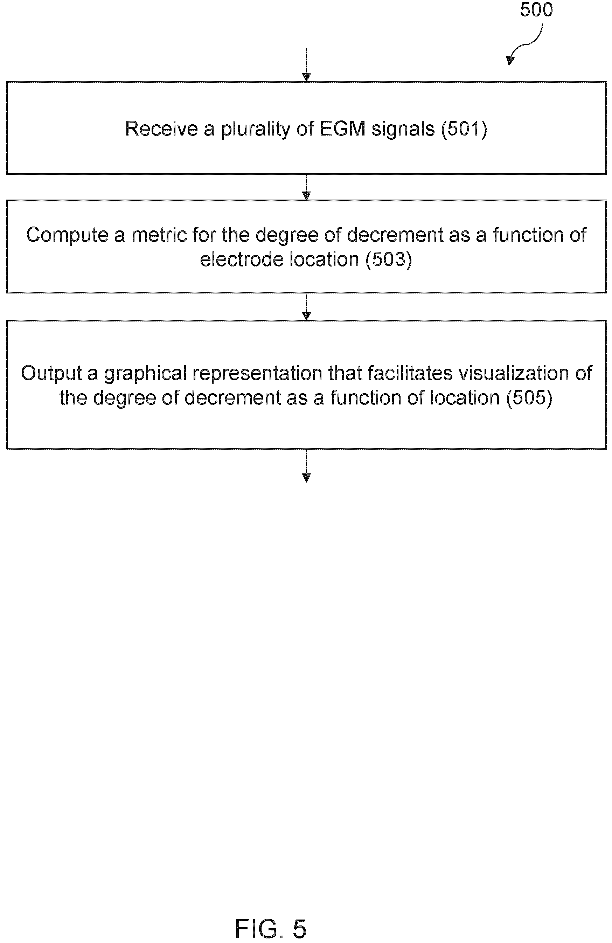

Accordingly, as shown in **FIG. 5****,** in embodiments of the disclosure system 8 and visualization and mapping module 58 may be configured to perform a method 500 and be configured to receive 501 a plurality of EGM signals, compute 503 a metric for the degree of decrement as a function of electrode location, and output 505 a graphical representation that facilitates visualization of the degree of decrement as a function of location.

Each EGM signal of the plurality of EGM signals comprises at least S1 and S2 stimuli.

The system 8 may also be configured to generate the S1 and S2 stimuli, for example, the signal generator 25 may generate the S1 and S2 stimuli. However, it will be understood that in other examples the S1 and S2 stimuli may be delivered by the electrophysiology system (e.g., Claris^{™}).

As described above, the system 8 may comprise an HD grid catheter, such as the catheter shown in FIG. 2. Accordingly, the system 8 and visualization and mapping module 58 may be configured to receive electrophysiological signals measured by a high-density grid catheter, and each EGM signal may correspond to a signal (for example, a unipolar, bipolar or an omnipolar signal).

The system 8 and mapping and visualization module 58 may be configured to obtain a template beat that contains both the S1 and S2 stimuli, and optionally the corresponding local EGM signals. The template beat may comprise a saved EGM signal of one cycle of, for example, ventricular tachycardia, VT. The template beat may be manually saved by a user, or may be obtained automatically by the system 8.

The system 8 and mapping and visualization module 58 may be configured to compute a metric for the degree of decrement by recognizing, by pattern matching, when the stimuli have been delivered. The system 8 and mapping and visualization module 58 may be configured to look to see if the current EGM signal matches the template beat based on a user defined matching score/threshold. If the correct coupling intervals are used, it is possible to fit both stimuli within S1S2 two beat waveform, pattern matching can be performed on that. In other words, if the correct coupling interval is selected, both S1 and S2 stimuli can be delivered within a single window of interest, such that morphological matching can be performed on an electrogram that includes both S1 and S2 stimuli, instead of having to do two matches on the S1 and S2 stimuli, respectively.

The system 8 and mapping and visualization module 58 may be configured to compute a metric for the degree of decrement by determining S1 and S2 EGM duration from each obtained EGM signal, and determining the degree of decrement based on an arithmetic function of the S1 and S2 duration.

The mapping and visualization module 58 may be configured to determine the S1 and S2 duration by any of the following methods (alone or in combination):
1. By determining the times from the start of the S1 signal to the end of the S1 signal, and start of the S2 signal to the end of the S2 signal (the end of the S2 signal may, for example, by the last deflection as shown in FIG. 3);
2. By determining when the EGM signals cross a selected voltage threshold;
3. By analysing the frequency of the EGM signal to determine the S1 and S2 duration; and
4. By analysing the energy distribution of the EGM signal - for example, timings may be derived based on the cumulative energy distribution. The energy distribution may be analysed in the wavelet domain (e.g., in the form of a scalogram) as opposed to in the time domain (e.g., an EGM). For example, a methodology may involve looking at the total energy of a signal within a defined window, and then annotate based on thresholds (e.g., 10% total energy and 90% total energy).

Examples of S1 and S2 EGM duration are also shown in FIG. 4 as described above.

The mapping and visualization module 58 may be configured to generate a colour map by interpolating the values of the obtained degree of decrement to the vertices of the 3D mapped geometry of the heart. The mapping and visualization module 58 may further be configured to scale the map according to the user's requirements so that areas of decrement (e.g., > about 10ms) are displayed as a specific colour on the 3D map.

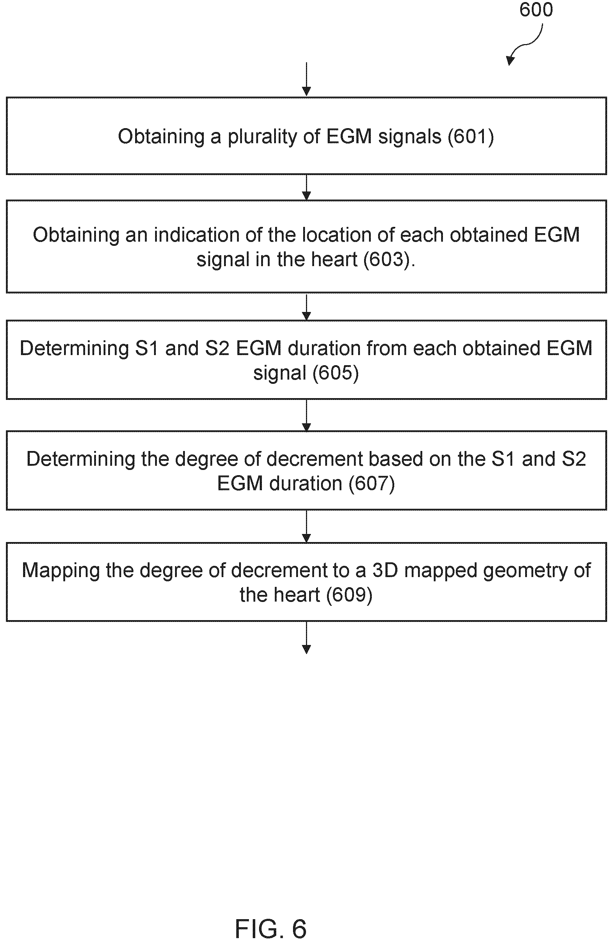

**FIG. 6** shows an example computer-implemented method 600 of decrement evoked potential mapping. The method comprises obtaining 601 a plurality of EGM signals, obtaining 603 an indication of the location of each obtained EGM signal in the heart (for example by using the system 8 and methodology described above with reference to FIG. 1 and FIG. 2), determining (605) S1 and S2 EGM durations from each obtained EGM signal, determining (607) the degree of decrement based on the determined S1 and S2 EGM durations; and mapping (609) the degree of decrement to a 3D mapped geometry of the heart.

Each EGM signal may comprise S1 and S2 stimuli. In some examples the method may further comprise delivering the S1 and S2 stimuli. The S2 stimulus may be at a user-selected coupling interval. Each EGM signal may be obtained between a corresponding plurality of electrode pairs. The electrode pairs may be on a grid-patterned electrode, such as an HD grid-patterned electrode. It will be understood that the EGM signals may be unipolar, bipolar or omnipolar.

Determining the degree of decrement based on the determined S1 and S2 EGM durations may comprise performing an arithmetic function of the S1 and S2 EGM durations, such as an arithmetic subtraction of the S2 duration from the S1 duration.

The method may further comprise mapping the geometry of the heart, for example using the method described above with reference to FIG. 1. The mapping may be performed using the same electrodes as the ones used for obtaining the EGM signals. Mapping the geometry of the heart may comprise forming a geometric model of the cardiac surface, and/or an electrophysiology, EP, map. It will be understood that forming an EP map is not required to form a geometric model of the heart.

In the case of forming an EP map, mapping the geometry of the heart may comprise receiving a plurality of electrophysiology, EP, data points at an electroanatomical mapping system (such as the system 8 shown in FIG. 1). The EP data points are a collection of electrophysiological signals and the locations at which they were measured, and may be used to create a geometric model, although only the location data is relevant to the geometry. The location data are treated as a point cloud, and there are various known techniques to generate a surface model from that point cloud.

The method may comprise computing, with the electroanatomical mapping system, a metric for each EP data point using the electrophysiological signals for the respective EP data point; and placing the corresponding electrophysiological signals on the geometry according to location (this may be performed with near one-to-one correspondence to map points; that is, less need to interpolate or project values) to create an electrophysiology map. The method may then comprise outputting, with the electroanatomical mapping system, a graphical representation that facilitates visualization of the heart based on the electrophysiological signals for the respective EP data point. It will be understood that the functions of receiving, computing and outputting described above may be performed by the mapping and visualization module 58.

The method may further comprise obtaining a template beat that contains both the S1 and S2 stiumuli and corresponding local EGM signals. Obtaining a plurality of EGM signals may comprise recognizing, by pattern matching, when the stimuli have been delivered. A template beat may comprise a saved EGM signal of one cycle of, for example, ventricular tachycardia. The template beat may be manually saved by a user, or may be obtained automatically by the system 8.

Obtaining an indication of the location of each obtained EGM signal in the heart may comprise obtaining an indication of the location of each obtained EGM signal in the heart at the time the S1 and S2 stimuli have been delivered. Obtaining an indication of the location may comprise determining the location of an electrode (for example using the mapping and visualization module 58), for example the electrode or electrode pair from which the EGM signal is obtained, at the time the S1 and S2 stimuli have been delivered.

Determining S1 and S2 EGM duration from each obtained EGM signal may comprise any of the following methods:
1. Determining the time from the start of the S1 signal to the end of the S2 beat (the end of the S2 beat may, for example, by the last deflection as shown in FIG. 3);
2. Determining when the EGM signals cross a selected voltage threshold;
3. Analysing the frequency of the EGM signal to determine the S1 and S2 duration; and
4. Analysing the energy distribution of the EGM signal.

Mapping the degree of decrement to a 3D mapped geometry of the heart may comprise mapping the degree of decrement to a 3D mapped geometry of the heart only in the event that the determined degree of decrement is above a selected threshold level degree of decrement. In this way a more user-friendly display of only the zone or zones of the heart with the highest degree of decrement may be shown to the user. The method may comprise generating a colour map by interpolating the values of the obtained degree of decrement to the vertices of the 3D mapped geometry of the heart.

The colour map may be scaled, for example according to the user's requirements, so that areas of decrement (e.g., > about 10ms) are displayed as a specific colour on the 3D map.

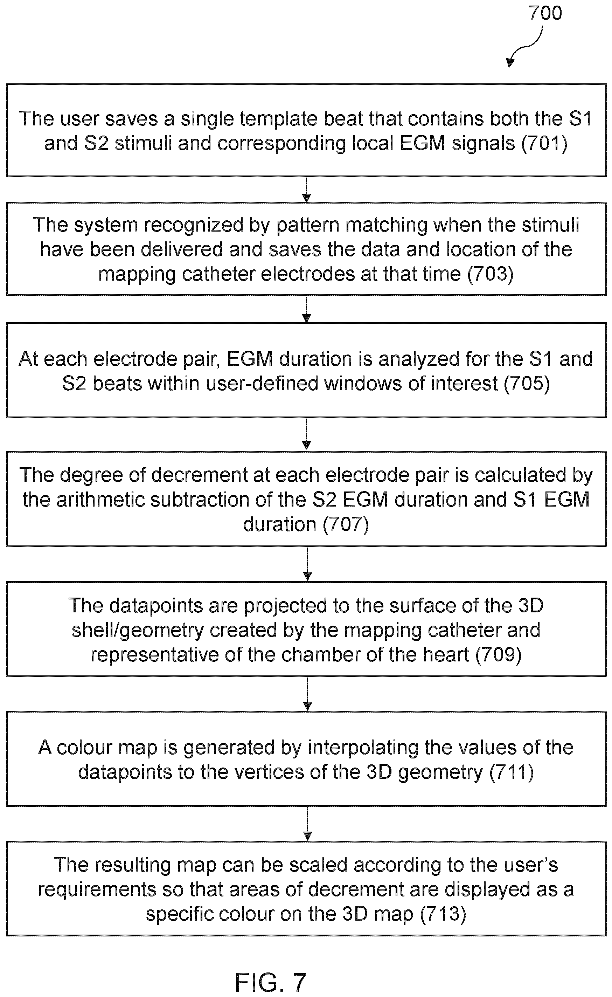

**FIG. 7** **shows** another example computer-implemented method 700 of decrement evoked potential mapping.
1. The user saves 701 a single template beat that contains both the S1 and S2 stimuli and corresponding local EGM signals.
2. During mapping, the system (e.g., electroanatomical mapping system 8) recognizes 703 by pattern matching when the stimuli have been delivered (and have captured correctly) and saves the data and location of the mapping catheter electrodes at that time.
3. At each electrode pair, EGM duration is analyzed 705 for the S1 and S2 beats within user-defined windows of interest. EGM duration may be measured in a number of ways. Optional manual reannotation of signals may be desirable in some circumstances.
4. The degree of decrement at each electrode pair is calculated 707 by the arithmetic subtraction of the S2 EGM and S1 EGM duration.
5. The datapoints (e.g., calculated degrees of decrement) are projected 709 to the surface of the 3D shell/geometry created by the mapping catheter and representative of the chamber of the heart (e.g., left ventricle).
6. A colour map is generated 711 by interpolating the values of the datapoints to the vertices of the 3D geometry.
7. The resulting map can be scaled 713 according to the user's requirements so that areas of decrement (e.g., > about 10ms) are displayed as a specific colour on the 3D map.

An advantage of the above system is that it may fully automate the DeEP mapping methodology, removing the requirement of physician assessment of electrograms in real time and provide more consistent measurements of EGM duration/decrement vs. manual approaches. Moreover, the location of the catheter for each S1-S2 pacing stimuli is recorded precisely at the time the pacing stimuli were delivered, reducing inaccuracies that may be introduced in current approaches using retrospectively labelled 3D tags.

The S1 and S2 durations may be analyzed or determined by any of the following methods:
1. By determining the time from the start of the S1 signal to the end of the S2 beat (the end of the S2 beat may, for example, by the last deflection as shown in FIG. 3);
2. By determining when the EGM signals cross a selected voltage threshold;
3. By analysing the frequency of the EGM signal to determine the S1 and S2 durations; and
4. By analysing the energy distribution of the EGM signal.

The utility of this invention may be in its integration into existing mapping systems, such as the EnSite^{™} mapping system platform, where it could be used by physicians during VT ablation procedures to generate a more accurate record of local decremental properties. This information could be used, in conjunction with other inputs, to define an appropriate ablation strategy.

It will be appreciated from the discussion above that the embodiments shown in the Figures are merely exemplary, and include features which may be generalised, removed or replaced as described herein and as set out in the claims.

Aspects of the present disclosure are described herein with reference to flowchart illustrations and/or block diagrams of methods, apparatuses (systems), and computer program products according to embodiments presented in this disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the block(s) of the flowchart illustrations and/or block diagrams.

These computer program instructions may also be stored in a computer readable medium that can direct a computer, other programmable data processing apparatus, or other device to function in a particular manner, such that the instructions stored in the computer readable medium produce an article of manufacture including instructions which implement the function/act specified in the block(s) of the flowchart illustrations and/or block diagrams.

The computer program instructions may also be loaded onto a computer, other programmable data processing apparatus, or other device to cause a series of operational steps to be performed on the computer, other programmable apparatus or other device to produce a computer implemented process such that the instructions which execute on the computer, other programmable data processing apparatus, or other device provide processes for implementing the functions/acts specified in the block(s) of the flowchart illustrations and/or block diagrams.

The flowchart illustrations and block diagrams in the Figures illustrate the architecture, functionality, and operation of possible implementations of systems, methods, and computer program products according to various embodiments. In this regard, each block in the flowchart illustrations or block diagrams may represent a module, segment, or portion of code, which comprises one or more executable instructions for implementing the specified logical function(s). It should also be noted that, in some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts, or combinations of special purpose hardware and computer instructions.

In the context of the present disclosure other examples and variations of the apparatus and methods described herein will be apparent to a person of skill in the art.

## Claims

1. A computer-implemented method of decrement evoked potential mapping, the method comprising:
obtaining a plurality of EGM signals, wherein each EGM signal comprises S1 and S2 stimuli;
obtaining an indication of the location of each obtained EGM signal in the heart;
determining S1 and S2 EGM durations from each obtained EGM signal;
determining the degree of decrement based on the determined S1 and S2 EGM durations; and
mapping the degree of decrement to a 3D mapped geometry of the heart.

2. The computer-implemented method of claim 1 further comprising mapping the geometry of the heart.

3. The computer-implemented method of claim 2, wherein mapping the geometry of the heart comprises forming an electrophysiology, EP, map:
receiving a plurality of EP data points at an electroanatomical mapping system, wherein each EP data point of the plurality of EP data points includes electrophysiological signals;
computing, with the electroanatomical mapping system, a metric for each EP data point using the electrophysiological signals for the respective EP data point; and
outputting, with the electroanatomical mapping system, a graphical representation that facilitates visualization of the heart based on the electrophysiological signals for the respective EP data point.

4. The computer-implemented method of claim 2 or 3 wherein obtaining a plurality of EGM signals comprises obtaining EGM signals using a grid-patterned electrode.

5. The computer-implemented method of any of the previous claims further comprising obtaining a template beat that contains both the S1 and S2 stimuli and corresponding local EGM signals; and
wherein obtaining a plurality of EGM signals comprises recognizing, by pattern matching, when the S1 and S2 stimuli have been delivered.

6. The computer-implemented method of claim 5 wherein obtaining an indication of the location of each obtained EGM signal in the heart comprises obtaining an indication of the location of each obtained EGM signal in the heart at the time the S1 and S2 stimuli have been delivered.

7. The computer-implemented method of any of the previous claims wherein determining S1 and S2 EGM duration from each obtained EGM signal comprises at least one of:
determining the time from the start of the S1 signal to the end of the S2 beat;
determining when the EGM signals cross a selected voltage threshold;
analysing the frequency of the EGM signal to determine the S1 and S2 durations; and
analysing the energy distribution of the EGM signal.

8. The computer-implemented method of any of the previous claims wherein mapping the degree of decrement to a 3D mapped geometry of the heart comprises mapping the degree of decrement to a 3D mapped geometry of the heart only in the event that the determined degree of decrement is above a selected threshold level degree of decrement.

9. The computer-implemented method of any of the previous claims further comprising generating a colour map by interpolating the values of the obtained degree of decrement to the vertices of the 3D mapped geometry of the heart.

10. The computer-implemented method of any of the previous claims wherein the degree of decrement is calculated by the arithmetic subtraction of the S2 EGM and S1 EGM durations.

11. The computer-implemented method of any of the previous claims further comprising applying the S1 and S2 extra-stimulus stimuli, wherein the S1 & S2 stimuli areat a user-selected coupling interval.

12. A system for decrement evoked potential, DeEP, mapping, the system comprising:
a mapping and visualization module configured to:
receive a plurality of EGM signals, wherein each EGM signal of the plurality of EGM signals comprises at least S1 and S2 stimuli;
compute a metric for the degree of decrement at each electrode pair as a function of electrode location; and
output a graphical representation that facilitates visualization of the degree of decrement as a function of location.

13. The system of claim 12 wherein the mapping and visualization module is configured to generate a colour map by interpolating the values of the obtained degree of decrement to the vertices of the 3D mapped geometry of the heart.

14. The computer-implemented method of any of the previous claims wherein the system is configured to applying the S1 and S2 extra-stimulus stimuli, wherein the S2 extra-stimulus is at a user-selected coupling interval.

15. A computer readable non-transitory storage medium comprising a program for a computer configured to cause a processor to perform the method of any of claims 1 to 11.
